# EUROPEAN PATENT APPLICATION

(11) **EP 4 741 796 A1**
(43) Date of publication of application: **13.05.2026**
(21) Application number: 24859068.9
(22) Date of filing: 27.05.2024
(51) Int. Cl.: G01N 1/32, C25F 3/02, G01N 1/28, G01N 33/202

(54) **METHOD FOR EXTRACTING DEPOSITS AND/OR INCLUSIONS**

(30) Priority: 31.08.2023 JP 2023140684
(71) Applicant: JFE Steel Corporation, Tokyo, 100-0011 (JP)
(72) Inventor: SUGAWARA, Seiya, Tokyo 100-0011 (JP)
(74) Representative: Grünecker Patent- und Rechtsanwälte PartG mbB
(86) International application number: PCT/JP2024/019344
(87) International publication number: WO 2025/047019

(57) **Abstract**

A metal material is electrolyzed while an electrolyte is flown. In this manner, precipitates and/or inclusions (precipitates and the like) in the metal material can be accurately extracted with ease. The electrolyzing is preferably performed while the electrolyte is flown along at least part of an electrolysis surface of the metal material, the electrolysis surface being a surface to contact the electrolyte. A flow velocity of the electrolyte flowing along the electrolysis surface is preferably not lower than 0.48 mm/s. It is preferable that the metal material and the electrolyte are stored in an electrolytic cell, and the electrolyte is injected into and discharged from the electrolytic cell, whereby the electrolyte is flown.

## Description

### TECHNICAL FIELD

The present invention relates to a method for extracting precipitates and/or inclusions.

### BACKGROUND ART

Precipitates and/or inclusions (hereinafter, also referred to as "precipitates and the like") present in a metal material such as a steel material affects properties of the metal material depending on the amount or the form thereof present in the material. Therefore, analysis of precipitates and the like is important in terms of property control of a metal material.

For analyzing precipitates and the like in a metal material, conventionally, the metal material has been electrolyzed using an electrolyte (Patent Literature 1). That is, a matrix element of a metal material (Fe in a case where a steel material is the metal material) is dissolved, whereby precipitates and the like are exposed on an electrolysis surface (surface in contact with an electrolyte) of the metal material, and the exposed precipitates and the like are observed or collected.

### CITATION LIST

### PATENT LITERATURE

Patent Literature 1: WO 2017/142084

### SUMMARY OF INVENTION

### TECHNICAL PROBLEMS

Using a steel material containing Ni and Mo in an amount of several mass percents as the metal material, the present inventor electrolyzed the metal material to extract precipitates and the like.

As a result, it was found that Fe, Ni, and Mo may form some compound (also referred to as "electrolysis product") on surfaces of the precipitates and the like.

In this case, precipitates and the like are not accurately extracted (as their intrinsic nature).

Since the electrolysis product formed on surfaces of precipitates and the like affects the analysis of the precipitates and the like, it is necessary to suppress formation of the electrolysis product.

In the meantime, for addressing a like problem, Patent Literature 1 discloses a technique of adding a chemical agent to an electrolyte, the chemical agent forming a complex with an element (hereinafter, also referred to as "inhibiting element") that inhibits accurate extraction of precipitates and the like.

When the technique described in Patent Literature 1 is adopted, however, it is necessary to prepare a suitable chemical agent for each inhibiting element (such as Fe, Ni, or Mo), involving much complexity. In addition, a suitable chemical agent cannot be prepared in some cases depending on the inhibiting element.

Therefore, desired is a method by which precipitates and the like can be extracted without preparing a chemical agent corresponding to an inhibiting element (i.e., with ease).

The present invention has been made in view of the foregoing, and an object of the invention is to provide a method by which precipitates and the like in a metal material can be accurately extracted with ease.

### SOLUTION TO PROBLEMS

The present inventor has made an intensive study to achieve the foregoing object.

As a result, the inventor found out the followings:
a) it is assumed that when a metal material is electrolyzed, an element (inhibiting element) constituting the metal material is dissolved into an electrolyte and undergoes some reaction, whereby an electrolysis product is formed on surfaces of precipitates and the like; and
b) in order to prevent occurrence of the above reaction, it suffices if the inhibiting element dissolved into the electrolyte is distanced from precipitates and the like exposed on the electrolysis surface of the metal material when the metal material is electrolyzed.

The present invention has been thus completed.

Specifically, the present invention provides the following [1] to [5].
[1] A method for extracting precipitates and/or inclusions to extract precipitates and/or inclusions in a metal material,
   the method comprising electrolyzing the metal material while flowing an electrolyte.
[2] The method for extracting precipitates and/or inclusions according to [1], wherein the electrolyzing is performed while the electrolyte is flown along at least part of an electrolysis surface of the metal material, the electrolysis surface being a surface to contact the electrolyte.
[3] The method for extracting precipitates and/or inclusions according to [2], wherein a flow velocity of the electrolyte flowing along the electrolysis surface is not lower than 0.48 mm/s.
[4] The method for extracting precipitates and/or inclusions according to any one of [1] to [3],
   wherein the metal material and the electrolyte are stored in an electrolytic cell, and
   the electrolyte is injected into and discharged from the electrolytic cell, whereby the electrolyte is flown.
[5] The method for extracting precipitates and/or inclusions according to any one of [1] to [3], wherein the precipitates and/or the inclusions exposed on a surface of the metal material are extracted after the electrolyzing.

### ADVANTAGEOUS EFFECTS OF INVENTION

According to the invention, precipitates and the like in a metal material can be accurately extracted with ease.

### BRIEF DESCRIPTION OF DRAWINGS

[FIG. 1] FIG. 1 is a schematic view showing an electrolysis system used for electrolysis of a metal material.
[FIG. 2A] FIG. 2A is a schematic view showing another example of the electrolysis system.
[FIG. 2B] FIG. 2B is a schematic view showing another example of the electrolysis system.
[FIG. 2C] FIG. 2C is a schematic view showing another example of the electrolysis system.
[FIG. 2D] FIG. 2D is a schematic view showing another example of the electrolysis system.
[FIG. 2E] FIG. 2E is a schematic view showing another example of the electrolysis system.
[FIG. 2F] FIG. 2F is a schematic view showing another example of the electrolysis system.
[FIG. 3] FIG. 3 is an SEM image showing residue in Inventive Example 1.
[FIG. 4] FIG. 4 is an SEM image showing residue in Comparative Example 1.

### DESCRIPTION OF EMBODIMENTS

The present embodiment will be described below.

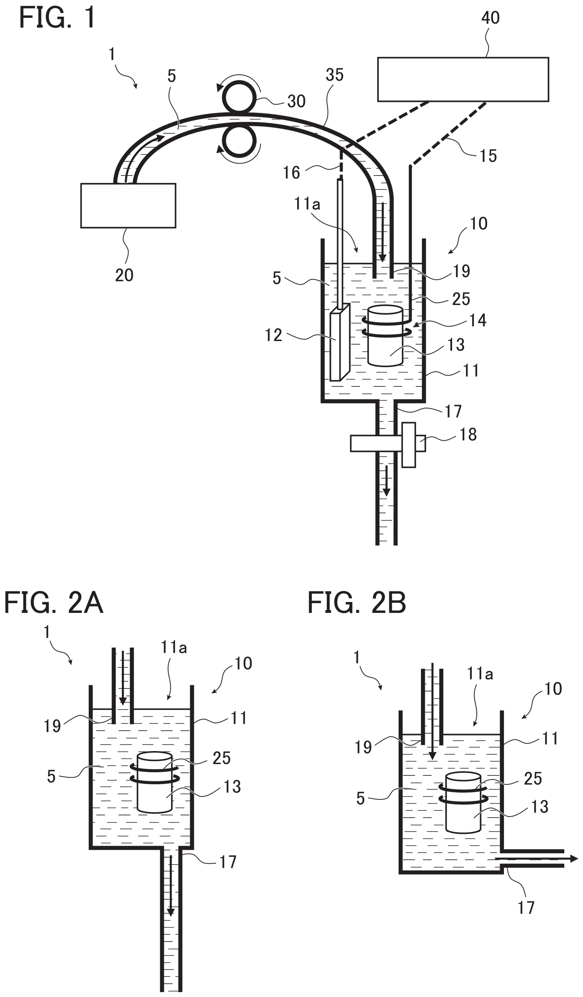

First, an electrolysis system 1 used for electrolysis of a metal material 13 in the present embodiment is described based on FIG. 1.

### <Outline of Electrolysis System>

FIG. 1 is a schematic view showing the electrolysis system 1 used for electrolysis of the metal material 13.

The electrolysis system 1 generally includes an electrolyzing apparatus 10, an electrolyte tank 20, a pump 30, a tube 35, and an electrochemical measurement device 40.

The electrolyzing apparatus 10 includes an electrolytic cell 11 having an opening portion 11a, and also includes a cathode 12 and an anode 14 constituted of the metal material 13.

An interior of the electrolytic cell 11 is filled with an electrolyte 5, and the cathode 12 and the anode 14 (metal material 13) are immersed in the electrolyte 5.

The metal material 13 shown in FIG. 1 is entirely immersed in the electrolyte 5, but this is not the sole case, and the metal material 13 only needs to be partly immersed in the electrolyte 5.

The respective components of the electrolysis system 1 are described below in more detail.

### <Metal Material and Anode>

The metal material 13 is not particularly limited as long as it is a metal material containing precipitates and/or inclusions (precipitates and the like), and is preferably a steel material.

In the field of steel, the amount and the form of precipitates and the like present in a steel material are precisely controlled to thereby impart a novel property to the steel material or improve the original properties of the steel material. It is hence important to accurately analyze precipitates and the like in a steel material.

An example of the steel material is a steel material containing several mass percents of Ni and Mo. When this steel material is electrolyzed, in addition to iron (Fe) being a matrix metal of the steel material, nickel (Ni) and molybdenum (Mo) are also dissolved into the electrolyte 5.

Precipitates and the like of the steel material include, for example, MnS and Cr compounds.

The shape of the metal material 13 is not particularly limited and is preferably a shape (such as a cylindrical shape or a cuboid shape) that does not interfere with the flow of the electrolyte 5, from the viewpoint of allowing the electrolyte 5 to flow along the electrolysis surface of the metal material 13.

The anode 14 is constituted of the metal material 13 and a lead wire 25 wound around the metal material 13. An example of a suitable material for the lead wire 25 is inert metal such as platinum (Pt). With the lead wire 25 being wound around the metal material 13, electrical conduction of the metal material 13 is assured while the metal material 13 is fixed.

The method for positionally fixing the metal material 13 is not limited to the above-described method, and, for example, when a specific surface (e.g., surface to be observed using an SEM after the electrolysis process) of the metal material 13 is electrolyzed, the lead wire 25 may be soldered on another surface than the specific surface of the metal material 13.

In addition, an alligator clip (not shown) connected to the lead wire 25 may be used to hold the metal material 13.

In a case where the metal material 13 is magnetic, in place of the lead wire 25, a tubular member (not shown) made of the same material as that of the lead wire 25 may be used.

The tubular member has one end surface being closed by a bottom plate and is placed inside the electrolytic cell 11 with the bottom plate facing downward. Along with this constitution, a neodymium magnet or another magnet is placed on the upper surface of the bottom plate (inner side of the tubular member), whereby the lower surface of the bottom plate (outer side of the tubular member) is adhered to the metal material 13. Accordingly, the metal material 13 can be positionally fixed in a hanging state.

In FIG. 1, since the metal material 13 is entirely immersed in the electrolyte 5, all of the non-winding region where the lead wire 25 is not wound (region not in contact with the wound lead wire 25) in the surface of the metal material 13 can be regarded as the electrolysis surface that is in contact with the electrolyte 5.

Of the surface of the metal material 13, any surface portion not requiring electrolysis may be covered using, for example, an insulating resin.

The anode 14 (more specifically, the lead wire 25 constituting the anode 14) is connected to the electrochemical measurement device 40 with use of a conductor wire 15.

### <Cathode>

As the cathode 12, various counter electrodes presently in practical use can be appropriately used.

Among those, platinum (Pt) is preferred because it is highly stable with respect to the electrolyte 5 and thus hardly contaminates the electrolyte 5.

The cathode 12 is connected to the electrochemical measurement device 40 with use of a conductor wire 16.

### <Electrochemical Measurement Device>

As the electrochemical measurement device 40, for instance, a potentiostat may be used in a case where the metal material 13 is electrolyzed while the potential of the metal material 13 is controlled, and a galvanostat may be used in a case where the metal material 13 is electrolyzed while the current in the metal material 13 is controlled. Use may be made of a potentiostat-galvanostat having both of their functions.

With the electrochemical measurement device 40 being driven, the potential or the current of the metal material 13 is controlled, and the metal material 13 is electrolyzed. The electrolysis conditions are not particularly limited, and a potential or a current at which precipitates and the like to be extracted do not decompose is preferred.

While the electrolysis system 1 shown in FIG. 1 adopts the two-electrode method, the electrolysis system 1 may also adopt the three-electrode method in which a reference electrode is used. The three-electrode method is preferred in terms of controlling.

### <Electrolyte Tank, Tube, and Pump>

In the electrolysis system 1, the electrolyte 5 is stored not only in the electrolytic cell 11 but also in the electrolyte tank 20.

One end of the tube 35 is connected to the electrolyte tank 20. The tube 35 is formed of a soft material such as silicone. The other end (inlet 19) of the tube 35 is disposed at the opening portion 11a of the electrolytic cell 11. That is, the electrolyte tank 20 and the electrolytic cell 11 are connected to each other via the tube 35.

The pump 30 is disposed in the middle of the tube 35. As the pump 30, a peristaltic pump is suitably used because it does not come in contact with the electrolyte 5, hardly causing contamination.

When the pump 30 is driven, the electrolyte 5 is sucked up from the electrolyte tank 20. The electrolyte 5 thus sucked up passes the interior of the tube 35 and is injected into the electrolytic cell 11 through the inlet 19, i.e., an end portion of the tube 35.

### <Electrolytic Cell>

As described above, the electrolytic cell 11 includes the opening portion 11a.

If the electrolytic cell 11 were a sealed system having no opening portion 11a, gas generated in the electrolysis process of the metal material 13 would be accumulated so that the pressure inside the electrolytic cell 11 would be positive, and injection of the electrolyte 5 would be possibly prevented. Therefore, the electrolytic cell 11 is preferably an open system having the opening portion 11a.

A tubular outlet 17 is provided at a position facing the inlet 19 on the opposite side from the opening portion 11a in the electrolytic cell 11. An openable-and-closable valve 18 is provided in the middle of the outlet 17. With the valve 18 being opened, the electrolyte 5 is discharged from the interior of the electrolytic cell 11.

### <Electrolyte>

The electrolyte 5 is not particularly limited, and an electrolyte having a conventionally known composition may be appropriately used. In the meantime, an electrolyte having a composition with which precipitates and the like of the metal material 13 do not dissolve is preferred. Specifically, suitable examples thereof include AA-based electrolyte (acetylacetone-tetramethylammonium chloride-methanol), MS-based electrolyte (4 vol% of methyl salicylate-1 mass% of salicylic acid-methanol), citric acid-based electrolyte (10 mass% of sodium citrate-1 mass% of potassium bromide-pure water-citric acid (for pH control)), or other electrolytes.

For instance, when the metal material 13 is a steel material, Fe dissolved into the electrolyte 5 may form an iron hydroxide or the like, and the outlet 17 may be clogged; in this case, a chemical agent that complexes Fe may be added to the electrolyte 5.

### <Electrolysis>

With the above-described configuration, when the electrochemical measurement device 40 is driven, the electrolysis surface (surface to contact the electrolyte 5) of the metal material 13 is electrolyzed, whereby precipitates and the like are exposed.

In a case where a steel material containing several mass percents of Ni and Mo is used as the metal material 13, Fe, Ni, and Mo constituting the material may be dissolved into the electrolyte 5 to serve as inhibiting elements, and may form an electrolysis product on surfaces of the exposed precipitates and the like.

Hence, the pump 30 is driven with the valve 18 being open in the electrolysis process of the metal material 13 in the present embodiment. The electrolyte 5 is then injected through the inlet 19 and, at the same time, is discharged from the outlet 17. That is, the electrolysis process of the metal material 13 is performed while the electrolyte 5 is flown.

Since the electrolyte 5 flows, even if an inhibiting element is dissolved into the electrolyte 5, the dissolved inhibiting element is distanced from precipitates and the like exposed on the electrolysis surface of the metal material 13.

In this manner, formation of an electrolysis product on surfaces of the precipitates and the like can be simply suppressed without using a chemical agent suitable for the inhibiting element.

The electrolyte 5 preferably flows along at least part of the electrolysis surface (e.g., surface to be observed using an SEM after the electrolysis process) of the metal material 13 in the present embodiment.

In other words, the respective portions such as the metal material 13, the inlet 19, and the outlet 17 are preferably arranged such that the electrolyte 5 flows along at least part of the electrolysis surface of the metal material 13.

For instance, the electrolyte 5 flows at least along a lateral surface of the cylindrical metal material 13 in the electrolysis system 1 shown in FIG. 1.

The flow velocity of the electrolyte 5 is determined by, for example, reaction which generates an electrolysis product (reaction of an inhibiting element dissolved into the electrolyte 5) and, hence, is appropriately set depending on the composition of the metal material or the like.

As the reaction time increases, an amount of the electrolysis product generated also increases.

In the meantime, when the flow velocity of the electrolyte 5 flowing along the electrolysis surface of the metal material 13 (e.g., lateral surface of the cylindrical metal material 13) is increased, an inhibiting element remains on this electrolysis surface for a shorter time, thereby reducing the reaction time.

Basically, a high flow velocity of the electrolyte 5 leads to an improved effect of distancing the inhibiting element from the precipitates and the like exposed on the electrolysis surface of the metal material 13.

Therefore, for example, when a steel material containing Ni and Mo is used as the metal material as described above, the flow velocity of the electrolyte 5 flowing along the electrolysis surface is preferably not lower than 0.48 mm/s, more preferably not lower than 0.65 mm/s, even more preferably not lower than 0.75 mm/s, and particularly preferably not lower than 1.33 mm/s. The upper limit is not particularly limited and is, for example, 2.00 mm/s.

The method for measuring the flow velocity of the electrolyte 5 is described later (see [Examples]).

The electrolyte 5 discharged to the outside of the electrolytic cell 11 through the outlet 17 may be returned to the electrolyte tank 20 and again injected into the electrolytic cell 11 through the inlet 19. In other words, the electrolyte 5 may be circulated.

In the meantime, the electrolyte 5 discharged from the outlet 17 contains an inhibiting element that has been dissolved from the metal material 13. Hence, from the viewpoint of distancing an inhibiting element from the electrolysis surface of the metal material 13, it is preferable that the electrolyte 5 is not circulated. In a case where the electrolyte 5 is circulated, an inhibiting element is preferably removed from the electrolyte 5 using, for example, an ion-exchange resin.

### <Extraction>

Thereafter, the precipitates and the like exposed on a surface of the metal material 13 are extracted by a known method.

For instance, the metal material 13 having been electrolyzed is immersed in alcohol such as methanol, and ultrasonic vibration is imparted to the metal material 13, whereby the exposed precipitates and the like are detached into the alcohol. The alcohol is then filtered using a filter (filter paper) to collect the precipitates and the like.

Here, observation of the precipitates and the like exposed on the surface of the metal material 13 with use of, for example, a scanning electron microscope (SEM), while the precipitates and the like are not detached from the metal material 13 and not collected, is also regarded as "extraction."

Formation of an electrolysis product on surfaces of the extracted precipitates and the like is suppressed. That is, the precipitates and the like can be accurately extracted (as their intrinsic nature) and can be hence precisely analyzed.

### <Another Exemplary Configuration of Electrolysis System>

FIGS. 2A to 2F are each a schematic view showing another example of the electrolysis system 1.

FIGS. 2A to 2F merely show the electrolytic cell 11, the inlet 19, the outlet 17, the metal material 13, and part of the lead wire 25, with other portions being omitted.

The configuration of the electrolysis system 1 used in the present embodiment is not limited to the configuration described based on FIG. 1 as long as the metal material 13 can be electrolyzed while the electrolyte 5 is flown.

For instance, the inlet 19 is disposed at a position facing the outlet 17 in the electrolysis system 1 described based on FIG. 1, but this is not the sole case, and the inlet 19 may be situated so as not to face the outlet 17 as shown in FIG. 2A.

In addition, the outlet 17 is disposed at a bottom surface of the electrolytic cell 11 in the electrolysis system 1 described based on FIG. 1, but this is not the sole case, and the outlet 17 may be disposed at a lateral surface of the electrolytic cell 11 as shown in FIG. 2B, for example.

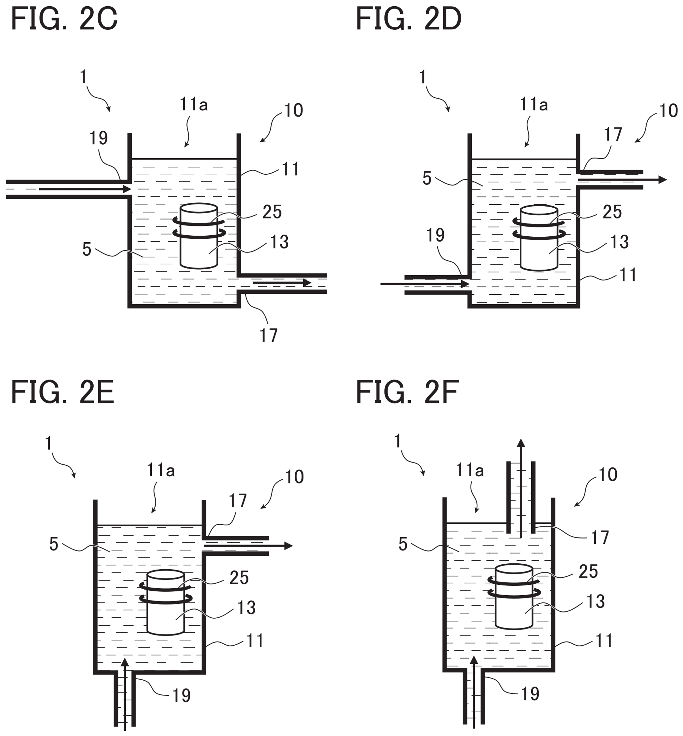

In addition, the inlet 19 is disposed at the opening portion 11a of the electrolytic cell 11 in the electrolysis system 1 described based on FIG. 1, but this is not the sole case, and a lateral surface of the electrolytic cell 11 may be provided with a hole to which the inlet 19 is connected as shown in FIGS. 2C and 2D, for example.

Moreover, for example, a bottom surface of the electrolytic cell 11 may be provided with a hole to which the inlet 19 is connected as shown in FIGS. 2E and 2F.

FIG. 1 and FIGS. 2A to 2F show the method (Method A) of discharging the electrolyte 5 through the outlet 17 while injecting the electrolyte 5 into the electrolytic cell 11 through the inlet 19.

The method for flowing the electrolyte 5 is not limited to this Method A, and, for example, use may be made of a method (Method B) in which the electrolyte 5 injected into the electrolytic cell 11 is rotated at a high velocity with a stirrer or the like, without being discharged through the outlet 17.

For instance, when precipitates and the like are exposed on the surface of the metal material 13 and directly observed with an SEM, a small electrolysis amount of the metal material 13 is mostly sufficient, and hence an amount of the inhibiting element dissolved into the electrolyte 5 is also small. In this case, even Method B can be expected to exhibit the above-described effect.

Meanwhile, when precipitates and the like exposed on the surface of the metal material 13 are collected and subjected to quantitative analysis, an electrolysis amount of the metal material 13 is relatively large, and hence Method A is preferred as the method for flowing the electrolyte 5, from the viewpoint of obtaining a sufficient effect described above.

### [EXAMPLES]

The invention is specifically described below by way of examples. However, the invention is not limited to the examples described below.

### <Inventive Example 1>

Using the electrolysis system 1 described based on FIG. 1, the metal material 13 was electrolyzed, and thereafter precipitates and the like were extracted as described below.

As the cathode 12, a Pt plate was used.

As the metal material 13 constituting the anode 14, a steel material in a cylindrical shape (diameter: 3.8 mm, length: 33.6 mm) containing 2.1 mass% of Ni and 1.1 mass% of Mo was used. The lead wire 25 made of Pt was wound around a lateral surface of the cylindrical metal material 13, thereby preparing the anode 14.

As the electrolyte 5, AA-based electrolyte (acetylacetone-tetramethylammonium chloride-methanol) was used. The acetylacetone content was 10 vol%. The tetramethylammonium chloride content in 100 mL of the electrolyte was 1 g.

As the electrolytic cell 11, a cylindrical container having the opening portion 11a in one surface was used. A hole was provided in a surface of the container opposite from the opening portion 11a, and a hose with the valve 18 was attached to the hole, thereby forming the outlet 17.

One end of the tube 35 made of silicone was connected to the electrolyte tank 20, and another end thereof was disposed at the opening portion 11a of the electrolytic cell 11 to serve as the inlet 19. More specifically, the inlet 19 was disposed at a position facing the outlet 17. The pump 30 being a peristaltic pump was disposed in the middle of the tube 35.

The cathode 12 and the anode 14 (metal material 13) were disposed inside the electrolytic cell 11, and the electrochemical measurement device 40 being a potentiostat-galvanostat was connected to them.

At this time, a straight line joining the inlet 19 and the outlet 17 was arranged to be parallel to the lateral surface of the cylindrical metal material 13 constituting the anode 14. In addition, the upper surface of the cylindrical metal material 13 was disposed directly below the inlet 19.

With the valve 18 of the outlet 17 being open, the pump 30 was driven.

Accordingly, the electrolyte 5 was injected into the electrolytic cell 11 through the inlet 19 and discharged through the outlet 17. By injecting and discharging the electrolyte 5 in this manner, the electrolyte 5 was flown along the lateral surface of the cylindrical metal material 13.

In this process, the flow velocity of the electrolyte 5 was 0.75 mm/s. The flow velocity was obtained by measuring a volume per unit time (unit: mm³/s) of the electrolyte 5 discharged through the outlet 17 and dividing the measured volume by the area (unit: mm²) of the bottom surface of the electrolytic cell 11.

In the meantime, separately, a float was placed in the electrolytic cell 11, and the flow velocity of the electrolyte 5 flowing along the lateral surface of the metal material 13 was determined; the result was similar to the above-described velocity.

The electrochemical measurement device 40 was driven in this state. The electrolysis surface of the metal material 13 was thus electrolyzed with the electrolyte 5 being flown. The dissolution (electrolysis) amount of the metal material 13 by the electrolysis process was about 0.02 g.

The metal material 13 having been electrolyzed was immersed in methanol, and ultrasonic vibration was imparted to the metal material 13. Thereafter, methanol was filtered with use of filter paper. As the filter paper, "Nuclepore membrane" (pore size: 0.2 µm) manufactured by Global Life Sciences Technologies Japan K.K. was used. Residue on the filter paper was observed using a scanning electron microscope (SEM).

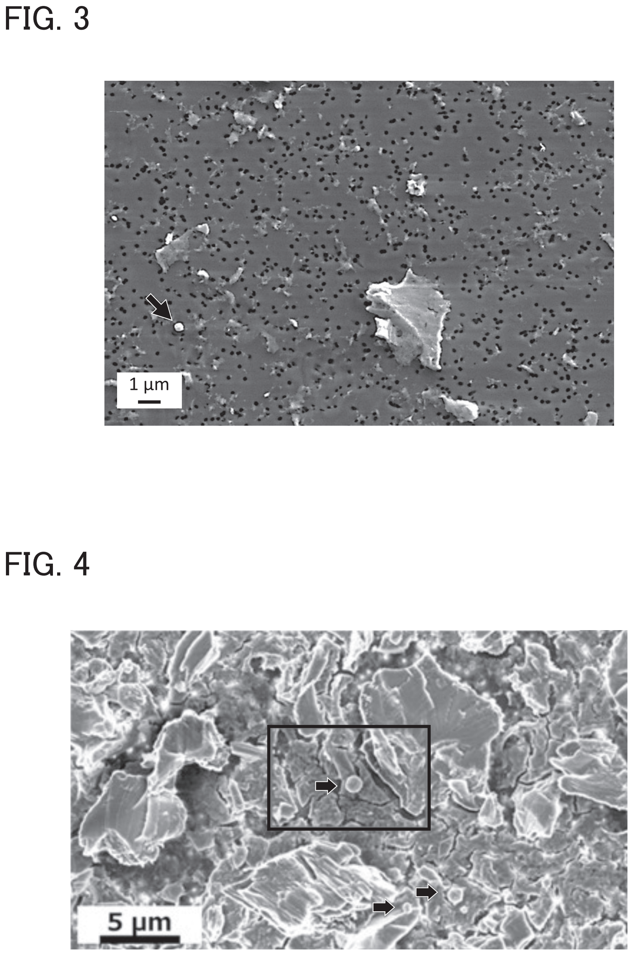

FIG. 3 is an SEM image showing residue in Inventive Example 1.

As shown by an arrow in FIG. 3, a precipitate or the like of spherical shape could be clearly confirmed in the SEM image. In addition, the spherical precipitate or the like was analyzed using an energy dispersive X-ray spectrometer (EDS) mounted on the SEM and found to be MnS.

In the analysis, no electrolysis product containing Fe, Ni, and Mo was observed on surfaces of the spherical precipitate and the like.

### <Comparative Example 1>

In Comparative Example 1, the metal material 13 was electrolyzed in the same manner as in Inventive Example 1 except that a commercial beaker was used as the electrolytic cell 11 so that the electrolyte 5 was not flown, and the electrolyte 5 was thereafter filtered using the filter paper.

FIG. 4 is an SEM image showing residue in Comparative Example 1.

As shown by arrows in FIG. 4, in addition to spherical precipitates and the like, a coating covering the spherical precipitates and the like was confirmed in the SEM image.

The spherical precipitates and the like were analyzed using the EDS mounted on the SEM, and it was confirmed that the precipitates and the like were MnS, and that the coating was a compound containing Fe, Ni, and Mo.

Since the compound was not found in an SEM image (not shown) obtained by observing the surface of the metal material 13 before the electrolysis process, the compound is presumably a compound (electrolysis product) generated by the electrolysis process.

As described above, it can be said that formation of an electrolysis product on surfaces of precipitates and the like was successfully suppressed in Inventive Example 1 where the metal material 13 was electrolyzed while the electrolyte 5 was flown.

### <Inventive Examples 2 to 5>

In Inventive Examples 2 to 5, the flow velocity of the electrolyte 5 was changed to 1.33 mm/s (Inventive Example 2), 0.48 mm/s (Inventive Example 3), 0.37 mm/s (Inventive Example 4), or 0.12 mm/s (Inventive Example 5). Except for these changes, the metal material 13 was electrolyzed in the same manner as in Inventive Example 1, and the filtering was thereafter performed using the filter paper.

Residues on the filter paper were observed using the SEM. As a result, a minute amount of electrolysis product was sometimes observed on surfaces of precipitates and the like in SEM images (not shown) of Inventive Examples 4 to 5. In SEM images (not shown) of Inventive Examples 2 to 3, on the other hand, no electrolysis product was observed on surfaces of precipitates and the like, as with Inventive Example 1.

### [REFERENCE SIGNS LIST]

- 1:: electrolysis system
- 5:: electrolyte
- 10:: electrolyzing apparatus
- 11:: electrolytic cell
- 11a: opening portion
- 12:: cathode
- 13:: metal material
- 14:: anode
- 15:: conductor wire
- 16:: conductor wire
- 17:: outlet
- 18:: valve
- 19:: inlet
- 20:: electrolyte tank
- 25:: lead wire
- 30:: pump
- 35:: tube
- 40:: electrochemical measurement device

## Claims

1. A method for extracting precipitates and/or inclusions to extract precipitates and/or inclusions in a metal material,
the method comprising electrolyzing the metal material while flowing an electrolyte.

2. The method for extracting precipitates and/or inclusions according to claim 1, wherein the electrolyzing is performed while the electrolyte is flown along at least part of an electrolysis surface of the metal material, the electrolysis surface being a surface to contact the electrolyte.

3. The method for extracting precipitates and/or inclusions according to claim 2, wherein a flow velocity of the electrolyte flowing along the electrolysis surface is not lower than 0.48 mm/s.

4. The method for extracting precipitates and/or inclusions according to any one of claims 1 to 3,
wherein the metal material and the electrolyte are stored in an electrolytic cell, and
the electrolyte is injected into and discharged from the electrolytic cell, whereby the electrolyte is flown.

5. The method for extracting precipitates and/or inclusions according to any one of claims 1 to 3, wherein the precipitates and/or the inclusions exposed on a surface of the metal material are extracted after the electrolyzing.
